# EUROPEAN PATENT APPLICATION

(11) **EP 4 737 560 A1**
(43) Date of publication of application: **06.05.2026**
(21) Application number: 24832465.9
(22) Date of filing: 27.06.2024
(51) Int. Cl.: C12N 5/0775, C12N 15/113, A61K 35/28, A61K 31/7105, A61P 11/00

(54) **MESENCHYMAL STEM CELL-DERIVED EXTRACELLULAR VESICLES AND USE THEREOF**

(30) Priority: 29.06.2023 KR 20230083905
(71) Applicant: CorestemChemon Inc., Seongnam-si, Gyeonggi-do 13486 (KR)
(72) Inventor: KIM, Kyung Suk, Seoul 02838 (KR); LEE, Tae Yong, Yongin-si Gyeonggi-do 17128 (KR)
(74) Representative: HGF
(86) International application number: PCT/KR2024/008989
(87) International publication number: WO 2025/005688

(57) **Abstract**

The present invention relates to human mesenchymal stem cell-derived extracellular vesicles and use thereof. The mesenchymal stem cell-derived extracellular vesicles according to the present invention inhibit the expression of fibrosis-related proteins, wound closure, and cell infiltration, which increase because of TGF-β1 treatment in lung epithelial cells. In addition, microRNAs, contained in large amounts in the extracellular vesicles, inhibit gene expression of fibrosis-related proteins in lung epithelial cells and lung fibroblasts. These extracellular vesicles significantly reduce, in lung fibrosis model mice, the expression of fibrosis-related proteins, collagen production and fibrosis regions. Therefore, the extracellular vesicles according to the present invention can be used to prevent and treat lung fibrosis.

## Description

### Technical Field

The present invention relates to a human mesenchymal stem cell-derived extracellular vesicle and a use thereof.

### Background Art

Idiopathic pulmonary fibrosis (IPF) is the most common disease of idiopathic interstitial pneumonia (IIP) within interstitial lung disease (ILD), but its exact cause remains unknown. Previously, chronic inflammation-induced fibrosis was considered the primary cause of idiopathic pulmonary fibrosis.

Pirfenidone and nintedanib, approved by the FDA in 2014, are used as therapeutic agents for idiopathic pulmonary fibrosis. However, these therapeutic agents do not address the underlying cause and have been reported to cause tolerability and side effects. In addition, drugs currently under development through clinical trials are single-acting therapeutic agents, raising concerns about efficacy and side effects. Therefore, the development of new therapeutic agents with multiple therapeutic mechanisms is needed.

Mesenchymal stem cells have been reported to be effective in treating idiopathic pulmonary fibrosis through their immunomodulatory, anti-inflammatory, and epithelial cell regeneration-promoting effects (Yang et al., Front Cell Dev Biol. (2021) 9:639657, Xie et al., Front Pharmacol. (2021) 11:590972). However, the biggest challenge with stem cells is that their engraftment and survival rates vary across patients, making it difficult to accurately demonstrate their efficacy. Furthermore, there is the potential for stem cells to transform into cancer cells. Therefore, research into treatments for pulmonary fibrosis utilizing mesenchymal stem cells is needed.

### Detailed Description of Invention

### Technical Problem

Accordingly, the present inventors conducted research to develop a therapeutic agent for pulmonary fibrosis. As a result, the present inventors found that extracellular vesicles isolated from a culture solution of mesenchymal stem cells exhibit an excellent effect in treating pulmonary fibrosis in a pulmonary fibrosis model mouse, thereby completing the present invention.

### Solution to Problem

In order to achieve the above object, in one aspect of the present invention, there is provided an extracellular vesicle comprising any one overexpressed microRNA selected from the group consisting of hsa-miR-148a-3p, hsa-miR-100-5p, hsa-miR-143-3p, hsa-let-7a-5p, hsa-let-7f-5p, and a combination thereof.

In another aspect of the present invention, there is provided a pharmaceutical composition for preventing or treating fibrosis, comprising the extracellular vesicle as an active ingredient.

In another aspect of the present invention, there is provided a use of the extracellular vesicle for the prevention or treatment of fibrosis.

In another aspect of the present invention, there is provided a method for preventing or treating fibrosis, comprising administering the extracellular vesicle to a subject.

### Effects of Invention

The mesenchymal stem cell-derived extracellular vesicles according to the present invention inhibited the expression of fibrosis-related proteins, wound closure, and cell infiltration increased by TGF-β1 treatment in lung epithelial cells. In addition, microRNAs, contained in large amounts in the extracellular vesicles, inhibited gene expression of fibrosis-related proteins in lung epithelial cells and lung fibroblasts. These extracellular vesicles significantly reduced, in pulmonary fibrosis model mice, the expression of fibrosis-related proteins, collagen production and fibrosis regions. Therefore, the extracellular vesicles according to the present invention can be used to prevent and treat pulmonary fibrosis.

### Brief Description of Drawings

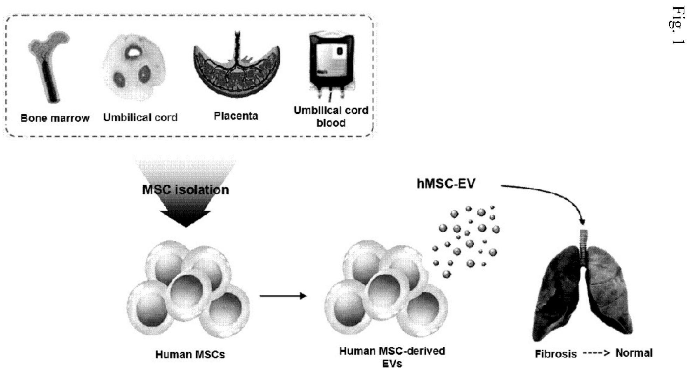

Figure 1 is a schematic diagram showing extracellular vesicles secreted from human mesenchymal stem cells (hMSCs) derived from various human tissues and their use in the treatment of pulmonary fibrosis.
Figure 2 is a schematic diagram showing a method for isolating human mesenchymal stem cell-derived extracellular vesicles according to one embodiment of the present invention.
Figure 3 is a graph and table showing the particle size distribution and concentration of extracellular vesicles (hMSCs-EVs) isolated from a culture solution of mesenchymal stem cells derived from human umbilical cord, using a nanoparticle tracking analyzer, according to one embodiment of the present invention.
Figure 4 is a diagram showing the results obtained by observing the double lipid membrane structure of hMSCs-EVs using a cryogenic transmission electron microscope, according to one embodiment of the present invention.
Figure 5 is a diagram showing the results obtained by identifying markers in hMSCs-EVs using Western blotting, according to one embodiment of the present invention.
Figure 6 is a graph showing the results obtained by identifying the phenotype of hMSCs-EVs using flow cytometry, according to one embodiment of the present invention.
Figures 7a and 7b are graphs showing the results obtained by analyzing the intrinsic factors of hMSCs-EVs using small RNA sequencing, according to one embodiment of the present invention.
Figure 8 is a graph showing the results of q-PCR identification of the gene expression of fibrosis-related factors after treating human lung epithelial cells (A549 cells) with TGF-β1 to induce EMT (epithelial-mesenchymal transition) and then treating them with hMSCs-EVs.
Figure 9 is a diagram showing the results obtained by identifying the wound healing effects after treating human lung epithelial cells (A549 cells) with TGF-β1 to induce EMT and then pre-treating them with hMSCs-EVs.
Figure 10 is a diagram and graph showing the results obtained by identifying the extent of cell migration and cell invasion after treating human lung epithelial cells (A549 cells) with TGF-β1 to induce EMT and then pre-treating them with hMSCs-EVs.
Figure 11 is a diagram showing the results obtained by identifying the expression of fibrosis-related proteins using Western blotting after treating human lung epithelial cells (A549 cells) with TGF-β1 to induce EMT and then pre-treating them with hMSCs-EVs.
Figure 12 is a diagram showing the results obtained by identifying the expression of fibronectin using cell immunofluorescence staining after treating human lung epithelial cells (A549 cells) with TGF-β1 to induce EMT and then pre-treating them with hMSCs-EVs.
Figure 13 is a graph showing the results obtained by identifying the expression of microRNA using q-PCR after overexpression of miR-148a-3p, miR-143-3p, miR-100-5p, Let-7a-5p, Let-7b-5p, or Let-7f-5p in human lung epithelial cells (A549 cells).
Figure 14 is a graph showing the results obtained by identifying the expression of microRNA using q-PCR after overexpression of miR-148a-3p, miR-143-3p, miR-100-5p, Let-7a-5p, Let-7b-5p, or Let-7f-5p in human lung fibroblasts (NHLF cells).
Figures 15a and 15b are graphs showing the results obtained by identifying the gene expression of fibrosis-related factors by treatment with TGF-β1 using q-PCR after overexpression of miR-148a-3p, miR-143-3p, miR-100-5p, Let-7a-5p, Let-7b-5p, or Let-7f-5p in human lung epithelial cells (A549 cells).
Figure 16 is a graph showing the results obtained by identifying the gene expression of fibrosis-related factors by treatment with TGF-β1 using q-PCR after overexpression of miR-148a-3p, miR-143-3p, miR-100-5p, Let-7a-5p, Let-7b-5p, or Let-7f-5p in human lung fibroblasts (NHLF cells).
Figure 17 is a diagram showing the results obtained by identifying the expression of fibrosis-related proteins by treatment with TGF-β1 using Western blotting after overexpression of miR-148a-3p, miR-143-3p, miR-100-5p, Let-7a-5p, Let-7b-5p, or Let-7f-5p in human lung epithelial cells (A549 cells).
Figure 18 is a diagram showing the results obtained by identifying the expression of fibrosis-related proteins by treatment with TGF-β1 using Western blotting after overexpression of miR-148a-3p, miR-143-3p, miR-100-5p, Let-7a-5p, Let-7b-5p, or Let-7f-5p in human lung fibroblasts (NHLF cells).
Figure 19 is a schematic diagram showing the experimental schedule for identifying the therapeutic effects of hMSCs-EVs on pulmonary fibrosis in a bleomycin-induced pulmonary fibrosis animal model (BLM-induced C57BL/6 mouse).
Figure 20 is a graph showing the results of q-PCR analysis of the gene expression of fibrosis-related factors in mouse lung tissue 8 days after a single administration of hMSCs-EVs in a bleomycin(BLM)-induced pulmonary fibrosis animal model.
Figure 21 is a graph showing the results obtained by measuring the concentration of soluble collagen in mouse lung tissue 8 days after a single administration of hMSCs-EVs in a bleomycin (BLM)-induced pulmonary fibrosis animal model.
Figure 22 is a diagram showing the results obtained by observing lung tissue using Masson's trichrome staining 8 days after a single administration of hMSCs-EVs in a bleomycin (BLM)-induced pulmonary fibrosis animal model. Magnification: 200x, Scale bar: 50 µm.
Figure 23 is a diagram showing the experimental schedule for identifying the therapeutic effects of hMSCs-EVs on pulmonary fibrosis in a bleomycin (BLM)-induced pulmonary fibrosis animal model.
Figure 24 is a diagram showing the results obtained by observing the morphology of the extracted mouse lung after administration of hMSCs-EVs in a bleomycin (BLM)-induced pulmonary fibrosis animal model.
Figure 25 is a graph showing the results obtained by identifying the gene expression of fibrosis-related factors in mouse lung tissue using q-PCR after administration of hMSCs-EVs in a bleomycin (BLM)-induced pulmonary fibrosis animal model.
Figure 26a is a graph showing the results obtained by identifying the expression of fibrosis-related proteins in mouse lung tissue using Western blotting after administration of hMSCs-EVs in a bleomycin (BLM)-induced pulmonary fibrosis animal model.
Figure 26b is a diagram showing the results obtained by identifying the expression of fibrosis-related proteins in mouse lung tissue using Western blotting after administration of hMSCs-EVs in a bleomycin (BLM)-induced pulmonary fibrosis animal model.
Figure 27 is a graph showing the results obtained by identifying the concentration of soluble collagen in mouse lung tissue after administration of hMSCs-EVs in a bleomycin (BLM)-induced pulmonary fibrosis animal model.
Figure 28 shows the results of hematoxylin and eosin staining (diagram) and Ashcroft score analysis (graph) of the pathological morphology of mouse lung tissue after administration of hMSCs-EVs in a bleomycin (BLM)-induced pulmonary fibrosis animal model.
Figure 29 is a diagram showing the results obtained by identifying the collagen areas in mouse lung tissue using Masson's trichrome staining after administration of hMSCs-EVs in a bleomycin (BLM)-induced pulmonary fibrosis animal model, and a graph showing the quantification thereof. X40: 40x magnification, X200: 200x magnification

### Best Mode for Carrying out the Invention

### Extracellular vesicle

In one aspect of the present invention, there is provided an extracellular vesicle comprising any one overexpressed microRNA selected from the group consisting of hsa-miR-148a-3p, hsa-miR-100-5p, hsa-miR-143-3p, hsa-let-7a-5p, hsa-let-7f-5p, and a combination thereof. At this time, the cell may be a human-derived cell, such as a cell derived from human tissue, a cell line, or a stem cell, and more specifically, may be a stem cell.

The cell derived from human tissue may be a cell derived from the human heart, stomach, large intestine, small intestine, lung, liver, kidney or uterus. In addition, the cell derived from human tissue may be a human derived immortalized cell line.

The stem cell may be a mesenchymal stem cell, a hematopoietic stem cell, a neural stem cell, an embryonic stem cell, or an induced pluripotent stem cell. Specifically, the stem cell may be a mesenchymal stem cell.

As used herein, the term "mesenchymal stem cell (MSC)" refers to stem cells that exist in cartilage, bone tissue, adipose tissue, the stroma of bone marrow, and the like, differentiated from the mesoderm formed by division of a fertilized egg, and may include mesenchymal stem cells of animals, including mammals, for example humans. Mesenchymal stem cells maintain stemness and self-renewal and have the ability to differentiate into various cells, including chondrocytes, osteoblasts, muscle cells, and adipocytes, and can be extracted from umbilical cord, bone marrow, adipose tissue, umbilical cord blood, synovial membrane, bone tissue (trabecular bone), muscle, infrapatellar fat pad, peripheral blood, liver, teeth, hair follicles, and the like. Mesenchymal stem cells are known to have immunomodulatory abilities that suppress the activity and proliferation of T cells and B cells, inhibit the activity of natural killer cells, and regulate the functions of dendritic cells and macrophages, and are therefore cells capable of allotransplantation and xenotransplantation.

The mesenchymal stem cell may be derived from umbilical cord, umbilical cord blood, bone marrow, fat, muscle, nerve, skin, amniotic membrane tooth, hair root cell, or placenta, or may be differentiated from an induced pluripotent stem cell, but is not limited thereto.

As used herein, the term "derived cell", for example, "derived mesenchymal stem cell", etc., refers to a cell substantially isolated from the tissue from which the cell originates, or a cell differentiated into an induced stem cell.

As used herein, the term "isolated extracellular vesicle" refers to an extracellular vesicle substantially isolated from a cell from which an extracellular vesicle originates, for example, a mesenchymal stem cell. Preferably, the extracellular vesicle may be isolated from a culture solution of mesenchymal stem cells. At this time, the medium used for culturing the cells may be any conventional medium known in the art to be suitable for cell culture. The medium may contain serum or may be a serum-free medium. Preferably, the medium may be a serum-free medium.

As used herein, the term "extracellular vesicle (EV)" refers to a nano-sized particle (about 50 nm to about 1,000 nm) packaged in a lipid bilayer and naturally secreted in living cells. The extracellular vesicle is composed of biologically active substances such as DNA, RNA, and proteins, and is known to play a role in information transfer between cells. In the present invention, the extracellular vesicles may include vesicles with a composition similar to that of extracellular vesicles (exosomes, microvesicles, multivesicles, and extracellular vesicle-like vesicles).

In the present invention, the extracellular vesicle may comprise any one overexpressed microRNA selected from the group consisting of hsa-miR-148a-3p, hsa-miR-100-5p, hsa-miR-143-3p, hsa-let-7a-5p, hsa-let-7f-5p, and a combination thereof.

As used herein, the term "microRNA (miRNA)" refers to a small RNA that controls gene expression in organisms. It is a small RNA comprising 20 to 25 nucleotides that can play an important role in regulating the gene expression by inhibiting target mRNA translation through complementary base pairing with the target mRNA 3'UTR (untranslated region). The microRNAs play a crucial role in cellular functions, including proliferation, differentiation, and apoptosis. They are evolutionarily conserved regulatory molecules present in all animals. Some microRNAs are known to regulate the gene expression through epigenetic regulatory mechanisms (histone modification, DNA methylation, etc.) associated with their promoter regions.

In one embodiment, the extracellular vesicle may comprise overexpressed hsa-miR-148a-3p. In one embodiment, the extracellular vesicle may comprise overexpressed hsa-miR-100-5p. In one embodiment, the extracellular vesicle may comprise overexpressed hsa-miR-143-3p. In one embodiment, the extracellular vesicle may comprise overexpressed hsa-let-7a-5p. In one embodiment, the extracellular vesicle may comprise overexpressed hsa-let-7f-5p.

In one embodiment, the extracellular vesicle may comprise overexpressed hsa-miR-148a-3p and hsa-miR-100-5p. In one embodiment, the extracellular vesicle may comprise overexpressed hsa-miR-148a-3p and hsa-miR-143-3p. In one embodiment, the extracellular vesicle may comprise overexpressed hsa-miR-148a-3p and hsa-let-7a-5p. In one embodiment, the extracellular vesicle may comprise overexpressed hsa-miR-148a-3p and hsa-let-7f-5p. In one embodiment, the extracellular vesicle may comprise overexpressed hsa-miR-100-5p and hsa-miR-143-3p. In one embodiment, the extracellular vesicle may comprise overexpressed hsa-miR-100-5p and hsa-let-7a-5p. In one embodiment, the extracellular vesicle may comprise overexpressed hsa-miR-100-5p and hsa-let-7f-5p. In one embodiment, the extracellular vesicle may comprise overexpressed hsa-miR-143-3p and hsa-let-7a-5p. In one embodiment, the extracellular vesicle may comprise overexpressed hsa-miR-143-3p and hsa-let-7f-5p. In one embodiment, the extracellular vesicle may comprise overexpressed hsa-let-7a-5p and hsa-let-7f-5p.

In one embodiment, the extracellular vesicle may comprise overexpressed hsa-miR-148a-3p, hsa-miR-100-5p, and hsa-miR-143-3p. In one embodiment, the extracellular vesicle may comprise overexpressed hsa-miR-148a-3p, hsa-miR-100-5p, and hsa-let-7a-5p. In one embodiment, the extracellular vesicle may comprise overexpressed hsa-miR-148a-3p, hsa-miR-100-5p, and hsa-let-7f-5p. In one embodiment, the extracellular vesicle may comprise overexpressed hsa-miR-148a-3p, hsa-miR-143-3p, and hsa-let-7a-5p. In one embodiment, the extracellular vesicle may comprise overexpressed hsa-miR-148a-3p, hsa-miR-143-3p, and hsa-let-7f-5p. In one embodiment, the extracellular vesicle may comprise overexpressed hsa-miR-148a-3p, hsa-let-7a-5p, and hsa-let-7f-5p. In one embodiment, the extracellular vesicle may comprise overexpressed hsa-miR-100-5p, hsa-miR-143-3p, and hsa-let-7a-5p. In one embodiment, the extracellular vesicle may comprise overexpressed hsa-miR-100-5p, hsa-miR-143-3p, and hsa-let-7f-5p. In one embodiment, the extracellular vesicle may comprise overexpressed hsa-miR-100-5p, hsa-let-7a-5p, and hsa-let-7f-5p. In one embodiment, the extracellular vesicle may comprise overexpressed hsa-miR-143-3p, hsa-let-7a-5p, and hsa-let-7f-5p.

In one embodiment, the extracellular vesicle may comprise overexpressed hsa-miR-148a-3p, hsa-miR-100-5p, hsa-miR-143-3p, and hsa-let-7a-5p. In one embodiment, the extracellular vesicle may comprise overexpressed hsa-miR-148a-3p, hsa-miR-100-5p, hsa-miR-143-3p, and hsa-let-7f-5p. In one embodiment, the extracellular vesicle may comprise overexpressed hsa-miR-148a-3p, hsa-miR-143-3p, hsa-let-7a-5p, and hsa-let-7f-5p. In one embodiment, the extracellular vesicle may comprise overexpressed hsa-miR-100-5p, hsa-miR-143-3p, hsa-let-7a-5p, and hsa-let-7f-5p.

In one embodiment, the extracellular vesicle may comprise overexpressed hsa-miR-148a-3p, hsa-miR-100-5p, hsa-miR-143-3p, hsa-let-7a-5p, and hsa-let-7f-5p.

At this time, the extracellular vesicle may comprise hsa-miR-148a-3p at about 1% to about 20% of the total microRNA content of the extracellular vesicle. Specifically, it may comprise at about 1% to about 20%, about 5% to about 18%, or about 10% to about 16%. Preferably, the extracellular vesicle may comprise hsa-miR-148a-3p at about 15.1%.

The extracellular vesicle may comprise hsa-miR-100-5p at about 1% to about 20% of the total microRNA content of the extracellular vesicle. Specifically, it may comprise at about 1% to about 20%, about 5% to about 17%, or about 10% to about 14%. Preferably, the extracellular vesicle may comprise hsa-miR-100-5p at about 11%.

The extracellular vesicle may comprise hsa-miR-143-3p at about 0.1% to about 10% of the total microRNA content of the extracellular vesicle. Specifically, it may comprise at about 0.1% to about 10%, about 0.5% to about 8%, or about 1% to about 6%. Preferably, the extracellular vesicle may comprise hsa-miR-143-3p at about 4.9%.

The extracellular vesicle may comprise hsa-let-7a-5p at about 0.1% to about 10% of the total microRNA content of the extracellular vesicle. Specifically, it may comprise at about 0.1% to about 10%, about 0.5% to about 8%, or about 1% to about 6%. Preferably, the extracellular vesicle may comprise hsa-let-7a-5p at about 4.6%.

The extracellular vesicle may comprise hsa-let-7f-5p at about 0.1% to about 10% of the total microRNA content of the extracellular vesicle. Specifically, it may comprise at about 0.1% to about 10%, about 0.5% to about 8%, or about 1% to about 6%. Preferably, the extracellular vesicle may comprise hsa-let-7f-5p at about 3.8%.

At this time, the microRNA may have fibrosis inhibition activity.

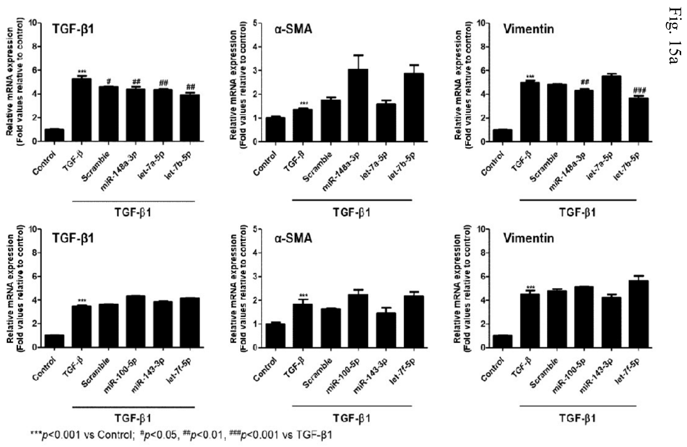

In one embodiment of the present invention, when lung epithelial cells or lung fibroblasts in which the microRNA is overexpressed were treated with TGF-β1, it was confirmed that the expression of genes and proteins related to epithelial-mesenchymal transition (EMT) and fibroblast myofibroblast transition (FMT) was reduced (Figures 15a, 15b, and 16 to 18).

In addition, the extracellular vesicle may further comprise any one expressed microRNA selected from the group consisting of hsa-miR-151a-3p, hsa-let-7b-5p, hsa-miR-21-5p, hsa-miR-10a-5p, hsa-miR-92a-3p, hsa-miR-486-5p, hsa-miR-99b-5p, hsa-let-7i-5p, hsa-miR-320a, hsa-miR-409-3p, hsa-miR-10b-5p, hsa-miR-127-3p, hsa-miR-26a-5p, hsa-miR-221-3p, hsa-let-7e-5p, hsa-miR-493-5p, hsa-miR-25-3p, hsa-miR-574-3p, hsa-miR-423-5p, hsa-miR-122-5p, hsa-miR-382-5p, hsa-miR-155-5p, hsa-miR-451a, hsa-miR-30a-3p, hsa-miR-28-3p, hsa-let-7g-5p, hsa-miR-379-5p, hsa-miR-23a-3p, hsa-miR-485-5p, hsa-miR-30a-5p, hsa-miR-30d-5p, hsa-miR-423-3p, hsa-miR-99a-5p, hsa-let-7c-5p, hsa-miR-92b-3p, hsa-miR-323a-3p, hsa-miR-574-5p, hsa-miR-197-3p, hsa-miR-432-5p, hsa-miR-140-3p, hsa-miR-370-3p, hsa-miR-196a-5p, hsa-miR-654-5p, hsa-miR-184, hsa-miR-181a-2-3p, and a combination thereof.

The particle size of the extracellular vesicle may be about 10 nm to about 1,000 nm. Specifically, the particle size of the extracellular vesicle may be about 10 nm to about 1,000 nm, about 20 nm to about 900 nm, about 30 nm to about 800 nm, about 40 nm to about 700 nm, about 50 nm to about 600 nm, or about 60 nm to about 500 nm.

### Pharmaceutical composition

In another aspect of the present invention, there is provided a pharmaceutical composition for preventing or treating fibrosis, comprising the extracellular vesicle as an active ingredient. The extracellular vesicle is the same as described above.

As used herein, the term "fibrosis" refers to the formation of excessive fibrous connective tissue in an organ or tissue. This can be distinguished from fibrous tissue, which is a normal component of the organ or tissue. Fibrosis can be understood as a fatal disease in which the excessive accumulation of extracellular matrix, such as fibronectin and collagen, by fibroblasts leads to the persistent loss of human tissue function due to the stiffening of organ tissue.

The fibrosis may occur, for example, in any one or more organs selected from the group consisting of the kidney, liver, lung, skin, heart, pancreas, urinary system, reproductive system, sweat gland, nerve, brain, bone marrow, muscle, and joint.

In the present invention, the fibrosis may be liver fibrosis, pulmonary fibrosis, skin fibrosis, articular fibrosis, nerve fibrosis, pancreatic fibrosis, renal fibrosis, muscle fibrosis, or peritoneal fibrosis. Specifically, the fibrosis may be pulmonary fibrosis, but is not limited thereto.

As used herein, the term "pulmonary fibrosis" refers to the development of scarred (fibrous) tissue due to the formation or development of excessive fibrous connective tissue (fibrosis) in the lungs. Specifically, the pulmonary fibrosis is a chronic disease that causes swelling and scarring of the alveoli and interstitial tissue of the lungs. This scar tissue replaces healthy tissue, causing inflammation, and chronic inflammation can be identified as a precursor to fibrosis. This damage to lung tissue can cause the lungs to stiffen and make it difficult for the subject to breathe on their own.

Specifically, the pulmonary fibrosis may include any one or more selected from the group consisting of idiopathic pulmonary fibrosis, radiation-induced lung injury, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, interstitial pulmonary fibrosis and diffuse pulmonary fibrosis, pulmonary edema, cystic fibrosis, and pulmonary fibrosis caused by metabolic diseases, but is not limited thereto.

In the present invention, the active ingredient of the pharmaceutical composition, an extracellular vesicle, may be included in any amount (effective amount) depending on the purpose, formulation, mixing purpose, and the like, as long as it can exhibit fibrosis inhibition activity. The typical effective amount of extracellular vesicle will be determined within the range of 0.001 wt% to 20.0 wt% based on the total weight of the composition. Here, the "effective amount" refers to an amount of the active ingredient capable of inducing a fibrosis inhibitory effect. Such an effective amount can be experimentally determined within the normal capabilities of those of ordinary skill in the art.

As used herein, the term "treatment" may be used to mean both therapeutic treatment and preventive treatment. At this time, prevention may be used to mean alleviating or reducing a pathological condition or disease in a subject. In one embodiment, the term "treatment" includes all applications or any form of medication for treating a disease in mammals, including humans. In addition, the above term includes inhibiting or slowing down a disease or the progression of a disease; restoring or repairing damaged or defective functions, thereby partially or completely alleviating a disease; or stimulating an inefficient process; or alleviating a serious disease.

As used herein, the term "efficacy" can be determined by one or more parameters, such as survival or disease-free survival over a period of time, such as 1 year, 5 years, or 10 years. In addition, the parameters can include suppression of the size of at least one tumor in a subject.

Pharmacokinetic parameters, such as bioavailability, and underlying parameters, such as clearance rate, can also affect efficacy. Therefore, "enhanced efficacy" (for example, improved efficacy) can be due to enhanced pharmacokinetic parameters and enhanced efficacy, and can be measured by comparing clearance rates and tumor growth in test animals or human subjects, or by comparing parameters such as survival, relapse rate, or disease-free survival.

The pharmaceutical composition of the present invention may be administered orally or parenterally (e.g., intravenously, subcutaneously, transdermally, nasally, or intratracheally), depending on the intended method, preferably parenterally. The dosage may vary depending on the patient's condition and body weight, the severity of the disease, the drug form, the route of administration, and the duration of administration, but can be appropriately selected by those of ordinary skill in the art.

The pharmaceutical composition according to the present invention is administered in a pharmaceutically effective amount. In the present invention, the pharmaceutically effective amount refers to an amount sufficient to treat a disease at a reasonable benefit/risk ratio applicable to medical treatments. The level of the effective dose may be determined according to factors including, the type and severity of the disease of the patient, the activity of the drug, the sensitivity to the drug, administration time, the route of administration and excretion rate, treatment period, the drug to be concurrently used, and other factors well known in the medical field. The composition according to the present invention may be administered as an individual therapeutic agent or in combination with another therapeutic agent, may be administered sequentially or simultaneously with a conventional therapeutic agent, and may be administered in single dose or in multiple doses. It is important to administer an amount that can achieve the maximum effect with the minimum amount without side effects by considering all of the above factors, and this can be easily determined by those of ordinary skill in the art.

Specifically, the preferred dosage of the pharmaceutical composition may vary depending on the patient's condition, body weight, sex, and age, the severity of the patient, and the route of administration. Typically, the dosage may be in the range of about 0.001 mg to about 150 mg per kg of body weight, preferably in the range of about 0.01 mg to about 100 mg per kg of body weight, which may be administered daily or every other day, or divided into 1 to 3 doses per day. However, the dosage may be increased or decreased depending on the route of administration, the severity of the disease, sex, body weight, age, and the like, and therefore should not be construed as limiting the scope of the present invention in any way.

The subject to which the pharmaceutical composition can be applied (prescribed) may be a mammal, and preferably, may be a human. In addition to the active ingredient, the pharmaceutical composition of the present invention may further comprise any compound or natural extract that has already been verified as safe and known to have fibrosis inhibition activity in order to increase and/or enhance the anti-fibrotic activity.

The pharmaceutical composition may further comprise a pharmaceutically acceptable carrier. The pharmaceutically acceptable carrier may be any carrier as long as it is non-toxic material suitable for delivery to a patient. Distilled water, alcohol, fats, waxes and inert solids may be included as a carrier. In addition, a pharmacologically acceptable adjuvant (buffering agent, dispersing agent) may be included in the pharmaceutical composition.

Specifically, the pharmaceutical composition may be prepared as a parenteral formulation according to the route of administration by a conventional method known in the art, including a pharmaceutically acceptable carrier in addition to the active ingredient. Here, "pharmaceutically acceptable" means that it does not inhibit the activity of the active ingredient and does not have toxicity beyond what the application (prescription) target can adapt.

When the pharmaceutical composition is prepared as a parenteral formulation, it may be formulated in the form of an injection, a transdermal preparation, a nasal inhalant, and a suppository together with a suitable carrier using methods known in the art. When it is formulated as an injection, as a suitable carrier, sterile water, ethanol, polyol such as glycerol or propylene glycol, or a mixture thereof may be used, and Ringer's solution, PBS (phosphate buffered saline) containing triethanolamine, or sterile water for injection, an isotonic solution such as 5% dextrose, and the like may be preferably used. Methods for formulating pharmaceutical compositions are well known in the art, and specific references can be made to Remington's Pharmaceutical Sciences (19th ed., 1995), which is incorporated herein by reference.

In another aspect of the present invention, there is provided a use of the extracellular vesicle or a pharmaceutical composition comprising the extracellular vesicle as an active ingredient for the prevention or treatment of fibrosis.

In another aspect of the present invention, there is provided a method for preventing or treating fibrosis, comprising administering to a subject the extracellular vesicle or a pharmaceutical composition comprising the extracellular vesicle as an active ingredient. Here, the extracellular vesicle, pharmaceutical composition, fibrosis, prevention, treatment, and administration are the same as described above. The subject may be a mammal, preferably a human. In addition, the subject may be a patient suffering from a disease or a subject at high risk of suffering from a disease.

In addition, the route of administration, dosage, and frequency of administration may vary depending on the patient's condition and the presence or absence of side effects, and it may be administered to the subject in various ways and amounts, and the optimal administration method, dosage, and frequency of administration can be selected within an appropriate range by those of ordinary skill in the art.

Specifically, the preferred dosage of the extracellular vesicle or the pharmaceutical composition may vary depending on the patient's condition, body weight, sex, and age, the severity of the patient, and the route of administration. Typically, the dosage may be in the range of about 0.001 mg to about 150 mg per kg of body weight, preferably in the range of about 0.01 mg to about 100 mg per kg of body weight, which may be administered daily or every other day, or divided into one to three doses per day. However, the dosage may be increased or decreased depending on the route of administration, the severity of the disease, sex, body weight, age, and the like, and thus, should not be construed as limiting the scope of the present invention in any way.

### Mode for Carrying out the Invention

Hereinafter, the present invention will be described in more detail by way of the following examples. However, the following examples are intended solely to illustrate the present invention, and the scope of the present invention is not limited to these examples.

### Example 1. Isolation of extracellular vesicles from mesenchymal stem cells

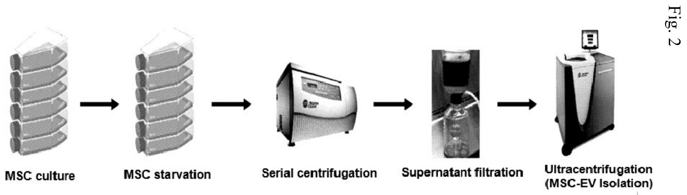

The extracellular vesicles were isolated from umbilical cord-derived mesenchymal stem cells using the process as shown in Figure 2.

Specifically, the human umbilical cord-derived mesenchymal stem cells were cultured in CSBM-A06 (Sigma Aldrich) culture medium containing 10% fetal bovine serum (Gibco, 16000-044), 1% penicillin/streptomycin (Gibco, 15140-122), and 1% GlutaMax (Gibco, 236320). After the completion of culturing, the mesenchymal stem cells were washed with Dulbecco's phosphate buffered saline (DPBS; Gibco, 14040-177) and replaced with medium containing 1% GlutaMax. Thereafter, the cells were cultured for 48 hours, and then the cell culture solution (culture supernatant) was collected. The reason for using serum-free medium was to prevent the mixing of FBS-derived extracellular vesicles with the extracellular vesicles secreted by stem cells, as fetal bovine serum (FBS) contains a significant amount of fetal bovine serum-derived extracellular vesicles.

The collected cell culture solution was centrifuged at 300xg for 10 minutes to remove any remaining cells. The resulting supernatant was re-centrifuged at 2,000xg for 20 minutes to remove cell debris and filtered through a 0.2 µm filter. Then, the filtrate was centrifuged in an ultracentrifuge at 100,000xg at 4 °C for 70 minutes. After ultracentrifugation, the supernatant was removed, and the extracellular vesicle pellet remaining in the lower layer was suspended in DPBS and re-centrifuged at 100,000xg at 4 °C for 70 minutes. The supernatant was removed, and the extracellular vesicle pellet remaining in the lower layer was diluted in normal saline (DAI HAN PHARM CO., LTD.) and used in the following experiments.

### Example 2. Comparison of physical and biochemical properties of mesenchymal stem cell-derived extracellular vesicles

The physical and biochemical properties of human mesenchymal stem cell-derived extracellular vesicles (hMSCs-EVs) were confirmed using a nanoparticle tracking analyzer, a cryogenic transmission electron microscope, Western blot, and a flow cytometer, using the same method as in Example 1 above.

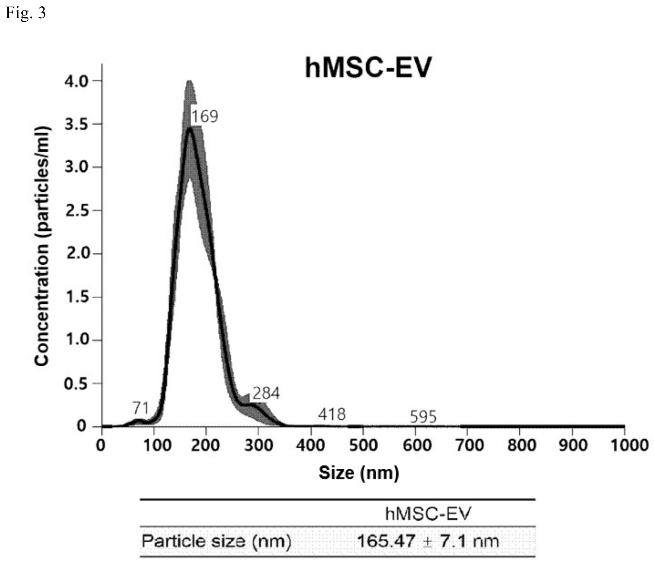

First, the size and particle count of the extracellular vesicles were measured using a nanoparticle tracking analyzer. As shown in Figure 3, it was confirmed that the extracellular vesicles had an average size of 200 nm or less.

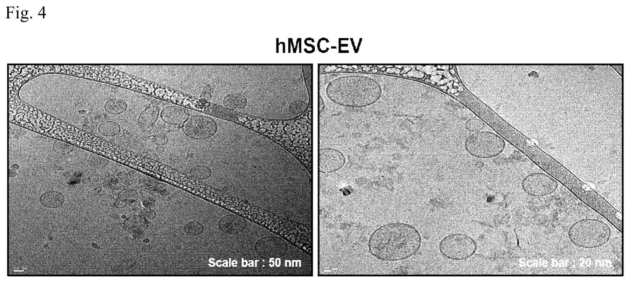

In addition, as shown in Figure 4, when observing the cell membrane using a cryogenic transmission electron microscope, a double phospholipid membrane structure was observed in the extracellular vesicles.

The expression of extracellular vesicle marker proteins was confirmed using Western blotting. At this time, antibodies used were anti-CD9 antibody (Santa Cruz, sc-13118), anti-CD63 antibody (Santa Cruz, sc-5275), anti-CD81 antibody (Santa Cruz, sc-7637), anti-GRP94 antibody (Santa Cruz, sc-32249), anti-β-actin antibody (Santa Cruz, sc-47778), or anti-Cytochrome C antibody (Santa Cruz, sc-13156).

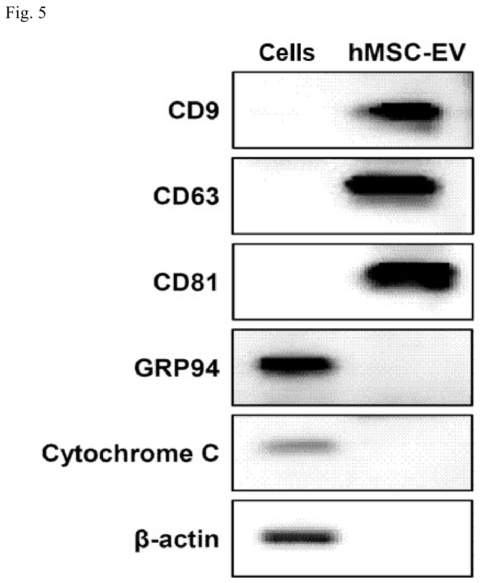

As a result, as shown in Figure 5, the expression of CD9, CD63, and CD81, marker proteins of extracellular vesicles (exosomes), was confirmed in the extracellular vesicle samples. On the other hand, GRP94, Cytochrome C, and β-actin (for purity confirmation), proteins known not to be expressed in extracellular vesicles, were expressed only in the cell samples.

In addition, the phenotype of extracellular vesicles was reconfirmed by a flow cytometer. Antibodies used were anti-CD9-PE antibody (BD, 555372), anti-CD63-PE antibody (BD, 556020), anti-CD81-APC antibody (BD, 551112), anti-GM130-PE antibody (Santa Cruz, sc55590), and anti-Calnexin-FITC antibody (Santa Cruz, sc-23954). PE-isotype IgG1 (BD Biosciences, 555749), APC-isotype IgG1 (BD Biosciences, 555751), or FITC-isotype IgG1 (BD Biosciences, 555748) were used as control antibodies, respectively.

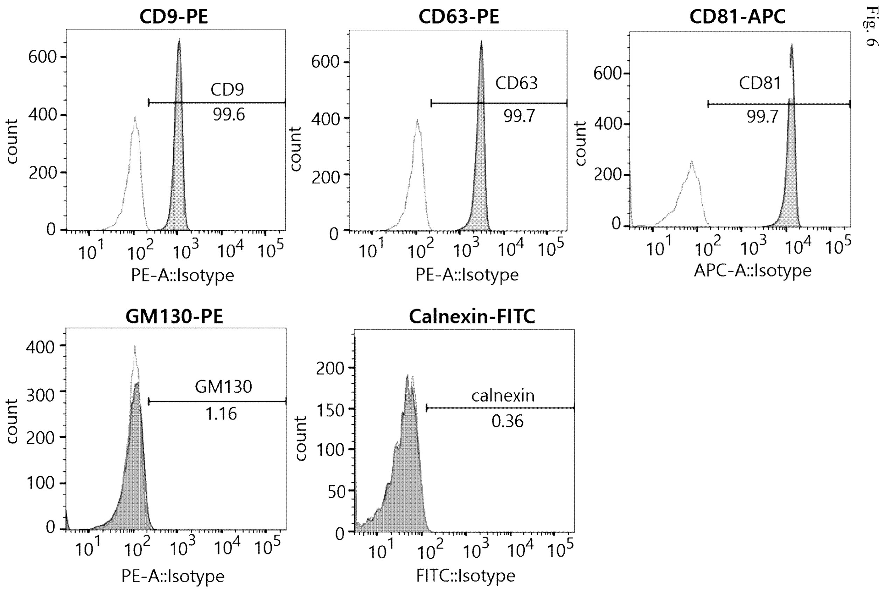

As a result, as shown in Figure 6, it was confirmed that the extracellular vesicle marker proteins CD9, CD63, and CD81 were expressed at 90% or more in the extracellular vesicle sample, while GM130 and Calnexin, proteins used to confirm purity, were expressed at less than 10%.

### Example 3. Isolation of extracellular vesicles from mesenchymal stem cells

In order to evaluate the intrinsic factors of the human mesenchymal stem cell-derived extracellular vesicles (hMSCs) isolated in Example 1 above, small RNA sequencing was performed to analyze the types and expression levels of microRNAs.

Specifically, RNA QC test was performed on the extracellular vesicles using the Bioanalyzer 2100 system. The total RNA was extracted according to the manufacturer's protocol, and a cDNA library was constructed from samples that passed the QC test and analyzed using next generation sequencing (NGS) technology. The results were used to conduct a comparative analysis of the entire microRNA profile.

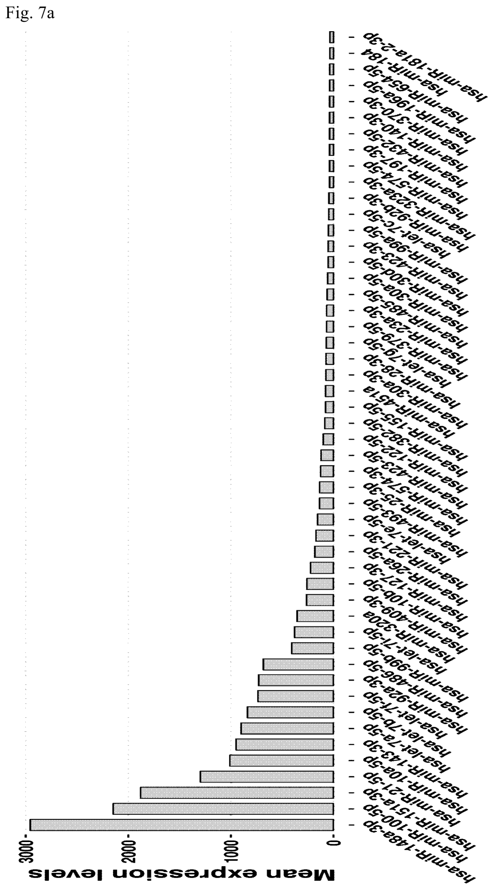

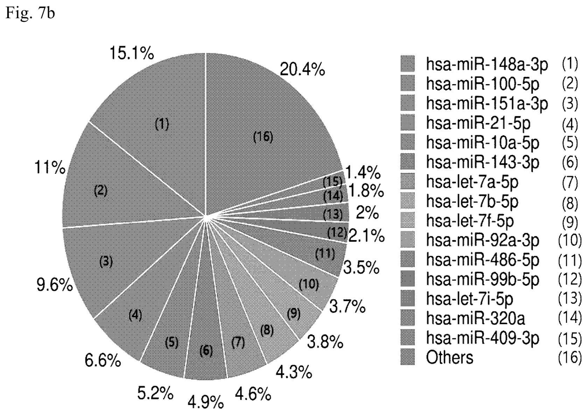

As a result, as shown in Figures 7a and 7b, it was confirmed that the extracellular vesicles contained 50 microRNAs. In particular, it was confirmed that among the microRNAs miR-148a-3p, let-7a-5p, let-7b-5p, miR-100-5p, miR-143-3p, and let-7f-5p were contained in large amounts.

### Example 4. In vitro evaluation of therapeutic efficacy of human mesenchymal stem cell-derived extracellular vesicles for pulmonary fibrosis

### Example 4.1. Analysis of gene expression of fibrosis-related factors

In order to confirm the inhibitory effect of the extracellular vesicles obtained according to the method described in Example 1 above on pulmonary fibrosis, an in vitro model similar to the pathogenesis of EMT and FMT using lung epithelial cells was constructed and the therapeutic efficacy for pulmonary fibrosis was evaluated.

Specifically, human lung epithelial cells (A549 cells) or human lung fibroblast cells (NHLF cells) were seeded into a 6 well plate at a concentration of 2 x 10⁵ cells/well and cultured for 24 hours under 5% CO₂, 37 °C conditions. Then, the cell culture medium was removed, washed twice with PBS, and treated with TGF-β1 (transforming growth factor β1) (10 ng/mL) and hMSCs-EVs (1 x 10⁸ particles/mL, 1 x 10⁹ particles/mL) prepared according to the method in Example 1. After an additional 48 hours of culture, the cell culture solution was removed and washed with PBS. RNA was extracted from the cells using the Easy-BLUE Total RNA Extraction kit (iNtRON Biotechnology) according to the manufacturer's protocol, and the RNA was quantified using a Nanodrop. cDNA synthesis was performed using Maxime RT Premix (Oligo dT primer) using the above RNA (1 µg) as a template in a nucleic acid amplifier according to the manufacturer's protocol. At this time, the nucleic acid amplifier was performed under the conditions of 45 °C for 60 minutes and 95 °C for 5 minutes. After the completion of reaction, the obtained cDNA was diluted 1/5 in DEPC-treated water (80 uL) and used as sample cDNA. The gene expression level of each sample was analyzed through q-PCR using respective primers for TGF-β1, fibronectin, α-SMA (smooth muscle actin), CTGF (connective tissue growth factor), COL3A1 (collagen type III alpha 1 chain), E-cadherin, and GAPDH. At this time, the primer sequences used are shown in Table 1.

**[Table 1]**

| Gene | Type | Primer sequence (5' to 3') | Species | SEQ ID NO |
|---|---|---|---|---|
| TGF-β1 | F | TACCTGAACCCGTGTTGCTCTC | human | 1 |
| | R | GTTGCTGAGGTATCGCCAGGA | | 2 |
| Vimentin | F | AGGCAAAGCAGGAGTCCACTGA | human | 3 |
| | R | ATCTGGCGTTCCAGGGACTCAT | | 4 |
| CTGF | F | CTTGCGAAGCTGACCTGGAAGA | human | 5 |
| | R | CCGTCGGTACATACTCCACAGA | | 6 |
| COL3A1 | F | GACCCTAACCAAGGATGCAA | human | 7 |
| | R | GGAAGTTCAGGATTGCCGTA | | 8 |
| E-cadherin | F | GCCTCCTGAAAAGAGAGTGGAAG | human | 9 |
| | R | TGGCAGTGTCTCTCCAAATCCG | | 10 |
| GAPDH | F | GTCTCCTCTGACTTCAACAGCG | human | 11 |
| | R | ACCACCCTGTTGCTGTAGCCAA | | 12 |
| COL1A1 | F | TCCTCCAGGGATCCAACGA | mouse | 13 |
| | R | GGCAGGCGGGAGGTCTT | | 14 |
| COL3A1 | F | GTGAAACTGGTGAACGTGGC | mouse | 15 |
| | R | ATAGGACCTGGATGCCCACT | | 16 |
| CTGF | F | GGGCCTCTTCTGCGATTTC | mouse | 17 |
| | R | ATCCAGGCAAGTGCATTGGTA | | 18 |
| Elastin | F | TTGCTGATCCTCTTGCTCAAC | mouse | 19 |
| | R | GCCCCTGGATAATAGACTCCAC | | 20 |
| RPL13A | F | CCCTCACCCTATGACAAGA | mouse | 21 |
| | R | CCTTTTCCTTCCGTTTCTCC | | 22 |
| Timp1 | F | GCAACTCGGACCTGGTCATAA | mouse | 23 |
| | R | CGGCCCGTGATGAGAAACT | | 24 |
| α-SMA | F | GTCCCAGACATCAGGGAGTAA | mouse | 25 |
| | R | TCGGATACTTCAGCGTCAGGA | | 26 |
| Fibronectin | F | TGACGCTGGCTTTAAGCTCA | mouse | 27 |
| | R | TCATCCGCTGGCCATTTTCT | | 28 |
| Fibronectin | F | ACAACACCGAGGTGACTGAGAC | human | 29 |
| | R | GGACACAACGATGCTTCCTGAG | | 30 |
| α-SMA | F | CTTTCTACAATGAGCTTCGTG | human | 31 |
| | R | ATTTGAGTCATTTTCTCCCG | | 32 |
| β-actin | F | TGGAATCCTGTGGCATCCATGAAAC | mouse | 33 |
| | R | TAAAACGCAGCTCAGTAACAGTCCG | | 34 |

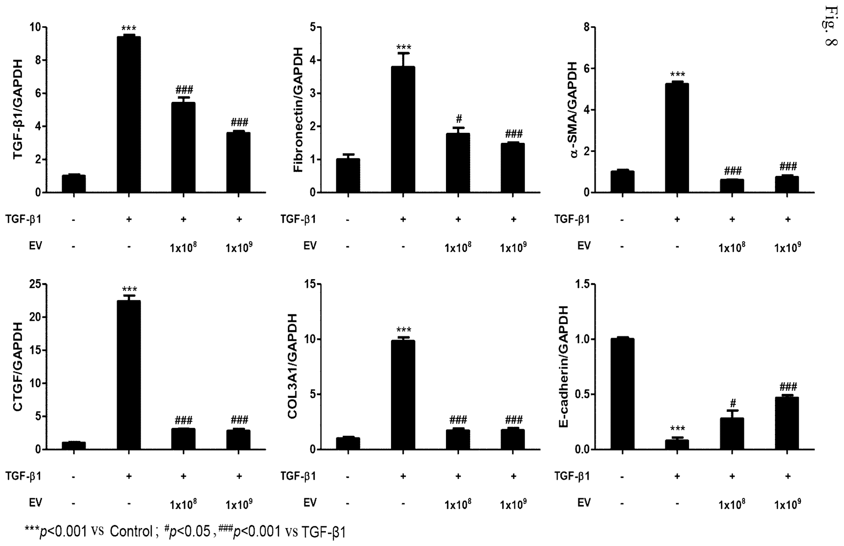

As a result, as shown in Figure 8, it was confirmed that the gene expression of fibrosis-related factors was significantly reduced in the TGF-β1+hMSCs-EV treatment group compared to the TGF-β1-only treatment group.

### Example 4.2. Analysis of wound closure effect

In order to confirm the epithelial-mesenchymal transition (EMT) inhibitory effect of the extracellular vesicles obtained according to the method described in Example 1 above, a wound closure experiment was performed.

Specifically, A549 cells were seeded into a 6 well plate at a concentration of 6 x 10⁵ cells/well and then cultured for 24 hours under 5% CO₂, 37 °C conditions. After 24 hours, a single cell layer was scratched using a 200 µL pipette tip to create a scratch. The cell culture solution was removed, and then the cells were washed twice with PBS and treated with TGF-β1 (10 ng/mL) and hMSCs-EVs (1 x 10⁸ particles/mL, 1 x 10⁹ particles/mL) prepared according to the method described in Example 1, followed by an additional 48-hour incubation. The cells were observed under a microscope and photographed during the incubation period (0, 24, and 48 hours).

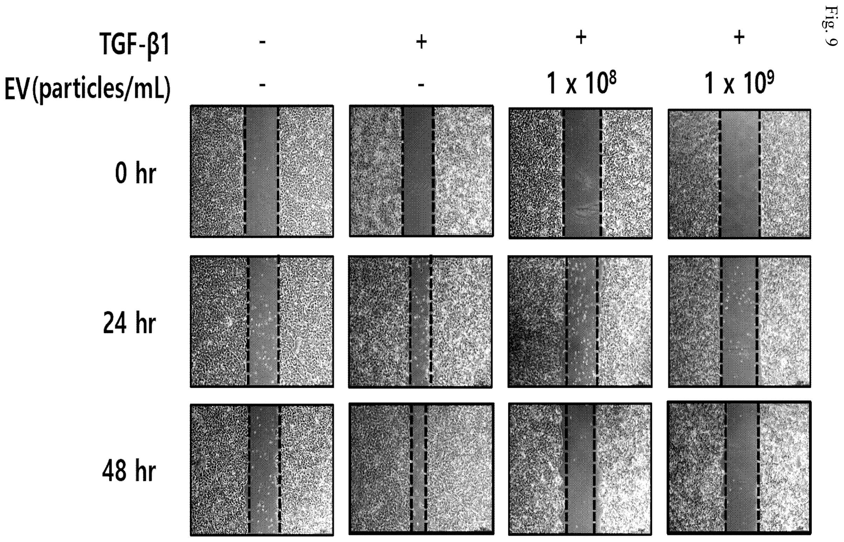

As a result, as shown in Figure 9, it was confirmed that wound closure was delayed in a concentration- and treatment time-dependent manner in the TGF-β1+hMSCs-EV treatment group compared to the TGF-β1-only treatment group.

### Example 4.3. Analysis of cell infiltration inhibitory effect

The cell invasion (migration and invasion) inhibitory effect of the extracellular vesicles obtained according to the method described in Example 1 above was confirmed.

Specifically, a 0.8 µm pore-sized Transwell (Corning) was used to confirm infiltration and migration. The cell medium used in the infiltration assay (migration assay) was prepared by treating serum-free DMEM/High glucose medium with TGF-β1 (10 ng/mL) and hMSCs-EVs (1 x 10⁸ particles/mL, 1 x 10⁹ particles/mL) according to the method described in Example 1. A549 cells were suspended in the medium prepared as described above at a concentration of 5 x 10⁴ cells/300 µL and loaded into the upper chamber of the Transwell. The lower chamber was loaded with DMEM/High glucose medium (700 µL) supplemented with 10% FBS, and then the two chambers were then joined. The cells were cultured for 24 hours under 5% CO₂, 37 °C conditions, fixed with 70% ethanol, and then stained with crystal violet solution. Then, the membrane of the upper chamber was wiped with a cotton swab and observed under a microscope to count the number of cells within the membrane.

The upper chamber of the transwell was coated with 100 uL (20 ug/well) of Matrigel, then the migration assay (invasion assay) was performed using the same method as the invasion assay.

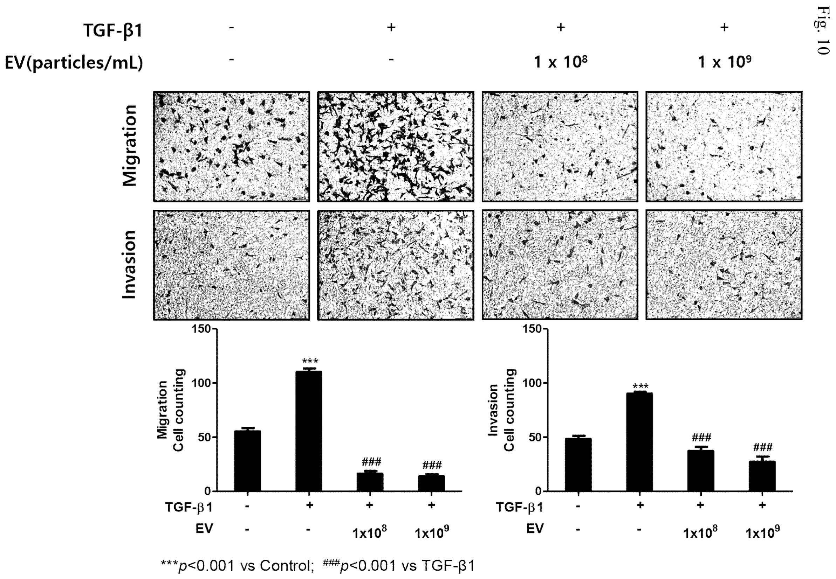

As a result, as shown in Figure 10, it was confirmed that cell migration and infiltration were reduced in the TGF-β+hMSCs-EV treatment group compared to the TGF-β-only treatment group.

### Example 4.4. Analysis of cell infiltration inhibitory effect

In order to confirm the mechanism of action of the extracellular vesicles obtained according to the method in Example 1 for the inhibition of epithelial-mesenchymal transition (EMT), the expression of fibrosis-related proteins was confirmed using Western blotting.

Specifically, A549 cells were seeded into a 6 well plate at a concentration of 2 x 10⁵ cells/well and then cultured for 24 hours under 5% CO₂, 37 °C conditions. The culture medium was removed and washed twice with PBS. The cells were then treated with TGF-β1 (10 ng/mL) and hMSCs-EVs (1 x 10⁸ particles/mL, 1 x 10⁹ particles/mL) prepared according to the method described in Example 1. After an additional 48 hours of culture, the culture medium was removed and washed with PBS. The cells were scraped with a scraper and collected in a 15 mL tube. After centrifugation, the supernatant was removed, and RIPA solution containing proteolytic enzyme inhibitors was added to the cell pellet and resuspended. The suspension was vortexed and reacted on ice for 30 minutes (vortexed every 10 minutes), and then centrifugation (13,000 rpm, 30 minutes, 4 °C) was performed. The supernatant was collected, and the proteins were quantified using the Bradford assay (BioRad).

The protein (30 µg) obtained through the above process was electrophoresed on a 10% SDS-PAGE gel and transferred to a PVDF (polyvinylidene difluoride) membrane. Thereafter, the membrane was blocked with blocking buffer (TBST buffer containing 5% nonfat dry milk) for 1 hour at room temperature, and then reacted overnight at 4 °C with the primary antibodies. At this time, the primary antibodies used were anti-TGFβ1 antibody (Abcam, ab92486), anti-fibronectin antibody (Abcam, ab2413), anti-COL1A1 antibody (Abcam, ab34710), anti-E-cadherin antibody (Cell Signaling, 3195S), anti-p-Smad2 antibody (Cell Signaling, 3108T), anti-Smad2 antibody (Cell Signaling, 5339T), or anti-β-actin antibody (Santa Cruz, sc-47778).

After the primary antibody reaction, washing was performed three times with TBST (Tris-buffered saline and Tween 20) solution, followed by reaction with the secondary antibodies for 1 hour at room temperature. The secondary antibodies used were anti-mouse antibody (Cell Signaling, 7076s) or anti-rabbit antibody (Cell Signaling, 7074s). Thereafter, the expression of proteins was confirmed by chemiluminescence imaging using ECL solution.

At this time, β-actin was used as an indicator to confirm the protein amount of each sample loaded in equal amounts. TGF-β1 was used as an EMT inducer, and TGF-β1, fibronectin, and COL1A1 were used as fibrosis markers. In addition, E-cadherin was used as an epithelial cell marker, and p-Smad2 and Smad2 were used as downstream signaling proteins involved in TGF-β-induced EMT.

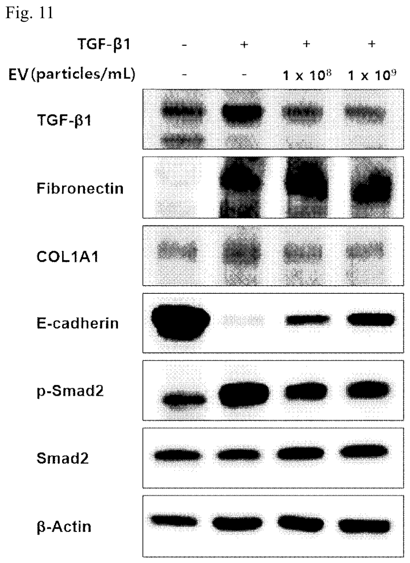

As a result, as shown in Figure 11, it was confirmed that the treatment with TGF-β1 increased the expression of mesenchymal cell markers, fibronectin, COL1A1, p-Smad2, and TGF-β1. On the other hand, it was confirmed that the treatment with extracellular vesicles reduced the expression of mesenchymal cell markers, which had been increased by TGF-β1, and increased the expression of E-cadherin, an epithelial cell marker.

### Example 4.5. Analysis of cell immunofluorescence staining

In order to confirm the mechanism of action of the extracellular vesicles obtained according to the method in Example 1 above for the inhibition of epithelial-mesenchymal transition (EMT), the expression of fibronectin was confirmed using cell immunofluorescence staining.

Specifically, A549 cells were seeded into a 6 well plate at a concentration of 2 x 10⁵ cells/well and then cultured for 24 hours under 5% CO₂, 37 °C conditions. The culture medium was removed and washed twice with PBS. The cells were then treated with TGF-β1 (10 ng/mL) and hMSCs-EVs (1 x 10⁸ particles/mL, 1 x 10⁹ particles/mL) according to the method described in Example 1. After an additional 48 hours of culture, the culture medium was removed and washed with PBS. The cells were fixed with 4% paraformaldehyde for 15 minutes and then washed three times with PBS. The blocking solution was added, and the blocking was performed at 60 rpm for 30 minutes. Thereafter, the cells washed three times with PBS. The reaction was performed overnight at 4 °C with primary antibody diluted at a ratio of 1:1000. At this time, the primary antibody used was anti-fibronectin antibody (Abcam, ab2413).

After the primary antibody reaction, the cells were washed three times with PBS and then reacted with the secondary antibody diluted at a ratio of 1:1000 for 1 hour at room temperature in a light-shielded state. The secondary antibody used was an anti-rabbit antibody (Invitrogen, A11008). Thereafter, the cells were washed three times with PBS, and nuclear staining was performed by adding DAPI fluorescent material (Thermo Scientific, 62248) and reacting for 5 minutes at room temperature in a light-shielded state. Thereafter, the cells were washed three times with PBS and observed under a fluorescence microscope.

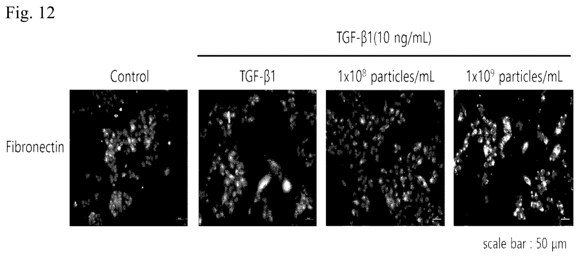

As a result, as shown in Figure 12, it was confirmed that the expression of fibronectin was reduced in the TGF-β1+hMSCs-EV treatment group compared to the TGF-β1 treatment group.

### Example 5. In vitro evaluation of therapeutic efficacy of increased microRNA expression for pulmonary fibrosis

### Example 5.1. Production of lung epithelial cells and lung fibroblasts with increased microRNA expression

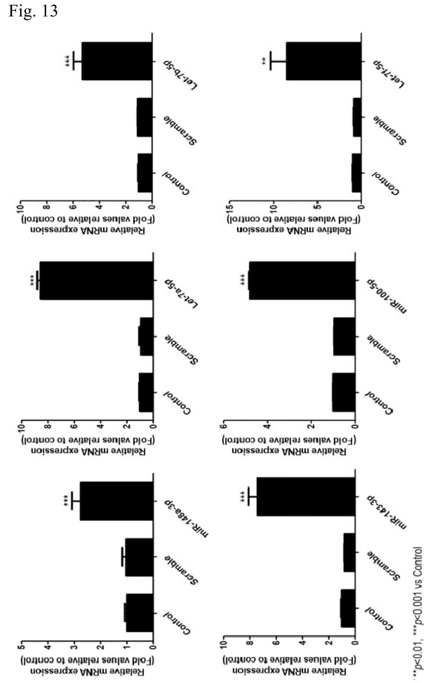

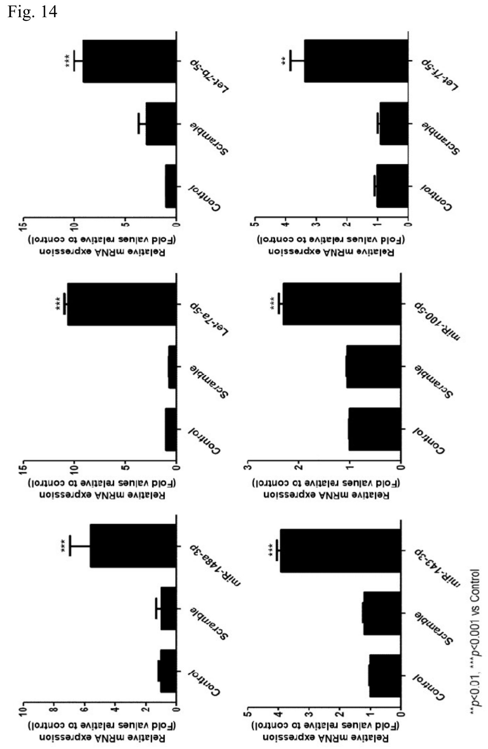

In order to confirm the therapeutic effects of increased microRNA expression on pulmonary fibrosis in lung epithelial cells or lung fibroblasts, A549 cells (Figure 13) and lung fibroblasts (NHLF) (Figure 14) were first transduced with microRNAs (miR-148a-3p (SEQ ID NO: 35), miR-100-5p (SEQ ID NO: 38), miR-143-3p (SEQ ID NO: 39), let-7a-5p (SEQ ID NO: 36), let-7b-5p (SEQ ID NO: 37), or let-7f-5p (SEQ ID NO: 40)), respectively. Thereafter, gene expression analysis was performed to confirm whether the transduced microRNAs were overexpressed.

Specifically, A549 cells, a human lung epithelial cell line, or normal human lung fibroblasts (NHLF cells) were seeded into a 6 well plate at a concentration of 1 x 10⁵ cells/well and then cultured for 24 hours under 5% CO₂, 37 °C conditions. Thereafter, the culture solution in each well was removed, and the cells were washed twice with PBS. Then, lentivirus (VectorBuilder) comprising each microRNA to be overexpressed was diluted in serum-free medium and applied to each well at an MOI of 10. After 6 hours, the culture solution in each well was removed, and the cells were washed twice with PBS. Thereafter, the cells were cultured in serum-supplemented medium at 37 °C in 5% CO₂ for 48 hours.

Thereafter, the cell culture solution in each well was removed, and the cells were washed twice with PBS, and then RNA was extracted. At this time, RNA was extracted using the miRNeasy Tissue/Cells Advanced Micro Kit (QIAGEN) according to the manufacturer's protocol, and the extracted RNA was quantified using a Nanodrop. cDNA synthesis was performed using the miRCURY LNA RT Kit (QIAGEN) using 200 ng of RNA in a nucleic acid amplifier according to the manufacturer's protocol (reaction at 40 °C for 60 minutes, at 95 °C for 5 minutes). The cDNA synthesized as described above was diluted at a ratio of 1/60 and used as a template cDNA. The gene expression was analyzed in a real-time nucleic acid amplifier using miRCURY LNA SYBR Green PCR Kit (QIAGEN) with miR-148a-3p, let-7a-5p, let-7b-5p, miR-100-5p, miR-143-3p, or let-7f-5p, and U6 primer (QIAGEN).

As a result, as shown in Figures 13 and 14, it was confirmed that the expression of each transfected microRNA was increased in A549 cells and NHLF cells in which each microRNA was overexpressed.

### Example 5.2. In vitro confirmation of changes in fibrosis-related gene expression by increased microRNA expression

A549 cells or NHLF cells, in which each microRNA was overexpressed, were treated with TGF-β1 (10 ng/mL) in the same manner as in Example 5.1 above. After 24 hours, the gene expression of TGF-β1, fibronectin, α-SMA, vimentin, CTGF (connective tissue growth factor), E-cadherin, or U6 was confirmed through q-PCR using the same method as in Example 4.1 above. At this time, the primer sequences used are shown in Table 1 above.

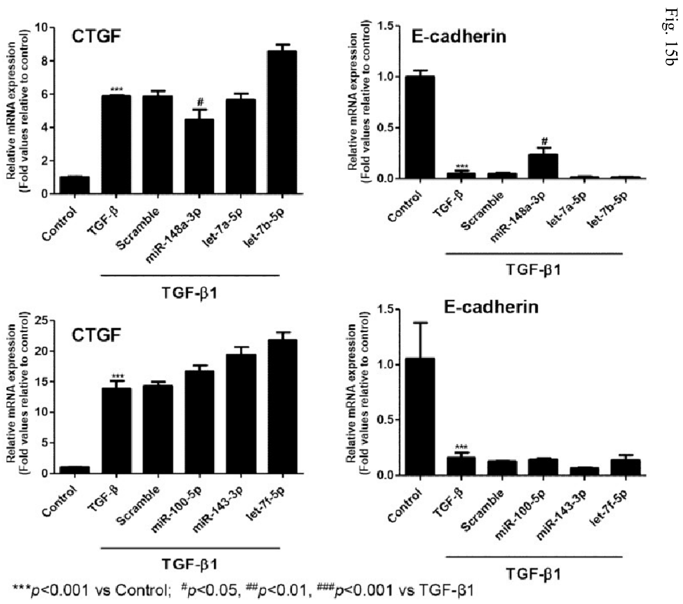

As a result, in the case of A549 cells, as shown in Figures 15a and 15b, the expression of TGF-β1, vimentin, and CTGF, which had been increased by TGF-β1 treatment, were reduced in miR-148a-3p overexpressing cells compared to the Scramble treatment group, while the expression of E-cadherin was increased. The expression of α-SMA was reduced in miR-143-3p-overexpressing cells, the expression of TGF-β1 was reduced in let-7a-5p-overexpressing cells, and the expression of TGF-β1 and vimentin were reduced in let-7b-5p-overexpressing cells.

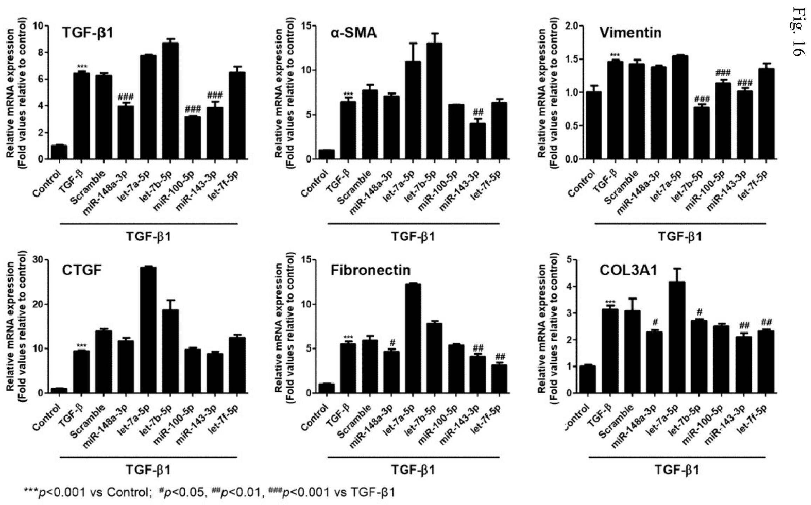

In the case of NHLF cells, as shown in Figure 16, the expression of TGF-β1, vimentin, fibronectin, and COL3A1, which had been increased by TGF-β1 treatment, was reduced in miR-148a-3p overexpressing cells compared to the Scramble treatment group, and the expression of TGF-β1, α-SMA, vimentin, and COL3A1 were reduced in miR-100-5p-overexpressing cells. In addition, the expression of TGF-β1, α-SMA, vimentin, CTGF, fibronectin and COL3A1 were reduced in miR-143-3p-overexpressing cells, and the expression of vimentin and COL3A1 were reduced in let-7b-5p-overexpressing cells. The expression of fibronectin and COL3A1 were reduced in let-7f-5p-overexpressing cells.

### Example 5.3. In vitro confirmation of changes in fibrosis-related protein expression by increased microRNA expression

A549 cells or NHLF cells, in which each microRNA was overexpressed, were treated with TGF-β1 (10 ng/mL) in the same manner as in Example 5.1 above. After 24 hours, the medium in each well was removed, washed with PBS, and each well was treated again with PBS (1 mL). The cells were collected using a scraper. Western blotting was performed on the cells obtained as described above using the same method as in Example 4.4 above.

At this time, the primary antibodies used were anti-TGFβ1 antibody (Abcam, ab92486), anti-vimentin antibody (Cell Signaling, 5741S), anti-CTGF antibody (Abcam, ab6992), anti-E-cadherin antibody (Cell Signaling, 3195S), anti-p-Smad2 antibody (Cell Signaling, 3108T), anti-Smad2 antibody (Cell Signaling, 5339T), or anti-β-actin antibody (Santa Cruz, sc-47778). TGF-β1 was used as the EMT inducer, and TGF-β1, α-SMA, CTGF, E-cadherin, and vimentin were used as evaluation markers. In addition, p-Smad2 was used to confirm whether TGF-β inhibited EMT downstream mechanisms.

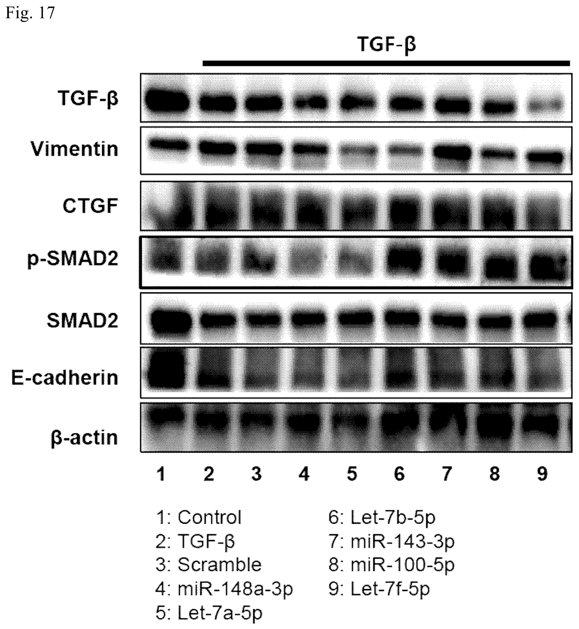

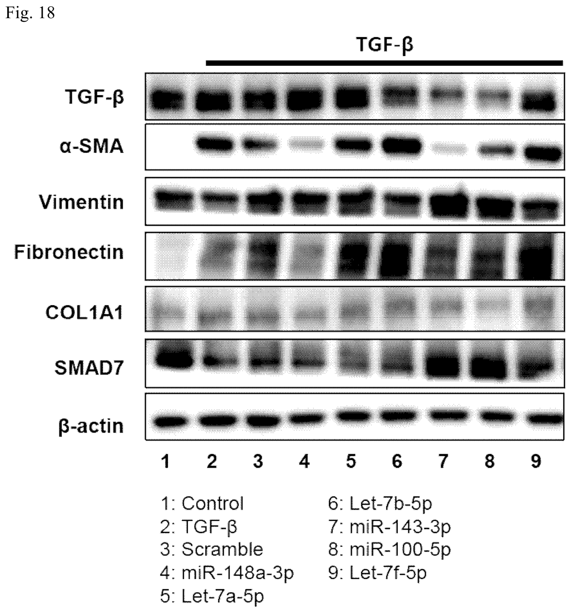

As a result, in the case of A549 cells, as shown in Figure 17 (A549), it was confirmed that the protein expression of TGF-β1 and vimentin were reduced in the cells overexpressing miR-148a-3p, let-7a-5p, let-7b-5p, miR-100-5p, or miR-143-3p compared to the Scramble treatment group. In addition, in the case of NHLF cells, as shown in Figure 18, it was confirmed that the protein expression of TGF-β1 and α-SMA were reduced in the cells overexpressing miR-148a-3p, miR-100-5p, or miR-143-3p compared to the Scramble treatment group. On the other hand, it was confirmed that the expression of SMAD7, which can inhibit the expression of TGF-β, was increased.

### Example 6. In vivo evaluation of therapeutic efficacy of human mesenchymal stem cell-derived extracellular vesicles for pulmonary fibrosis

### Example 6.1. Evaluation of therapeutic efficacy for pulmonary fibrosis in an animal model of pulmonary fibrosis produced by intraperitoneal administration of bleomycin

### Example 6.1.1. Construction of animal model of pulmonary fibrosis and administration of extracellular vesicles

In order to confirm the therapeutic effect of the extracellular vesicles obtained according to the method described in Example 1 above on pulmonary fibrosis, an animal model of pulmonary fibrosis was produced and the therapeutic effect of the extracellular vesicles on pulmonary fibrosis was evaluated.

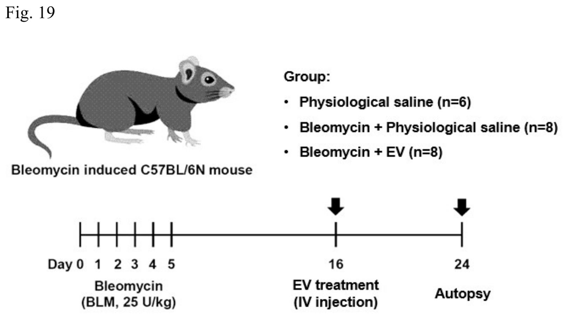

Specifically, as shown in the animal experiment schematic diagram in Figure 19, 8-week-old male C57BL/6 mice (25 g) were intraperitoneally administered with bleomycin (BLM), known to induce pulmonary fibrosis, at a concentration of 25 units/kg (100 µL) once daily for a total of 6 days to produce a pulmonary fibrosis model. The extracellular vesicles to be used in the experiment were prepared by dispersing the extracellular vesicles obtained according to the method described in Example 1 above in physiological saline solution so that they could be administered at a concentration of 2 x 10¹⁰ particles/mouse, thereby preparing an injection.

The extracellular vesicles (2 x 10¹⁰ particles/mouse) prepared as described above were administered intravenously as a single dose of 100 µL per mouse to the pulmonary fibrosis model mice (day 16 after the start of bleomycin administration). During the administration and observation period, general symptoms and body weight were observed once a day and recorded for each animal for 8 days. On day 8 after administration of extracellular vesicles (day 24 after the start of bleomycin administration), lung tissue was collected from the mice and subjected to a soluble collagen assay and Masson's trichrome staining to confirm the therapeutic effect of extracellular vesicles on pulmonary fibrosis.

### Example 6.1.2. Confirmation of changes in gene expression of fibrosis-related factors in lung tissue

Lung tissue was obtained from the experimental mice of Example 6.1.1 above, and the gene expression of fibrosis-related factors in lung tissue was confirmed using q-PCR.

Specifically, the right middle lobe of the lung tissue from the experimental mice of Example 6.1.1 above was extracted and pulverized using a homogenizer. Trizol solution (1 mL) was added to the pulverized tissue samples and reacted overnight at -80 °C. Then, RNA was extracted using an RNA extraction kit. After measuring the RNA concentration, cDNA was synthesized according to the manufacturer's protocol. The gene expression levels in each sample were confirmed through q-PCR using primers for COL1A1 (collagen type I alpha 1 chain), COL3A1, CTGF, elastin, and RPL13A (ribosomal protein L13a). At this time, the primer sequences used are shown in Table 1.

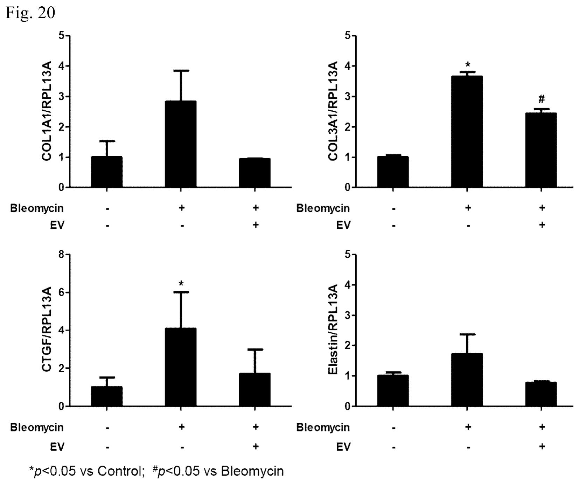

As a result, as shown in Figure 20, it was confirmed that the gene expression of COL1A1, COL3A1, CTGF, and elastin was increased by bleomycin (BLM) administration, and that the increased gene expression of the above genes was reduced by BLM+hMSCs-EV administration.

### Example 6.1.3. Confirmation of collagen deposition in lung tissue using soluble collagen assay

The lung tissue was obtained from the experimental mice of Example 6.1.1 above, and the concentration of collagen present in the lung tissue was measured using a soluble collagen assay.

Specifically, the right middle lobe of the lung obtained from the experimental mice of Example 6.1.1 above was extracted, and the extracted tissue was pulverized using a homogenizer. The pulverized tissue was treated with a pepsin-ascorbic acid solution and reacted overnight at 4 °C. Then, centrifugation was performed at 3,000 xg for 10 minutes, and then the supernatant was collected. The supernatant (100 µL) was mixed with Sircol dye reagent (1 µL) and reacted at room temperature for 30 minutes with rocking. After the reaction was completed, centrifugation was performed at 13,000 x g for 10 minutes, the supernatant was removed, and acid-salt wash reagent (750 uL) was added and mixed by vortexing. The mixture was centrifuged again at 13,000xg for 10 minutes, the supernatant was removed, alkali reagent (1 uL) was added and mixed by vortexing. The mixture (200 uL) was transferred to a 96-well plate, and the absorbance (556 nm) was measured using a microplate reader.

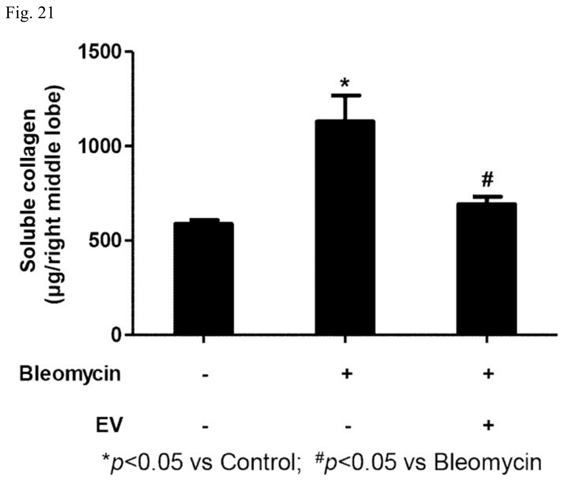

As a result, as shown in Figure 21, it was confirmed that bleomycin (BLM) administration increased the collagen concentration in lung tissue, but BLM+hMSCs-EV administration significantly reduced the collagen concentration.

### Example 6.1.4. Confirmation of collagen deposition in lung tissue using Masson's trichrome staining

The collagen deposition in the lung tissue obtained from the experimental mice of Example 6.1.1 above was confirmed using Masson's trichrome staining.

Specifically, the left lobe of the lung tissue obtained from the experimental mice of Example 6.1.1 above was extracted and fixed in a 4% paraformaldehyde solution. The fixed mouse lung tissue was then sectioned into 4 µm-thick paraffin tissue sections.

The paraffin was removed from the tissue sections, hydrated, and then washed with water. The washed tissue sections were treated with Bouin's solution, refixed at 56 °C for 1 hour, and then washed in running water for 5-10 minutes. The tissue sections were then stained with Weigert's iron hematoxylin solution for 10 minutes, washed in running water for 10 minutes, and then washed again in distilled water. Next, the tissue sections were treated with Biebrich scarlet-acid fuchsin solution, stained for 10-15 minutes, and then washed again in distilled water. The sections were reacted in a phosphomolybdic-phosphotungstic acid solution for 10-15 minutes and then reacted in an aniline blue solution for another 5-10 minutes. The stained tissue sections were washed with distilled water and then reacted in a 1% acetic acid solution for 2-5 minutes. The sections were then dehydrated in 95% ethanol, treated with a xylene solution, mounted with a mounting solution, and observed under a microscope.

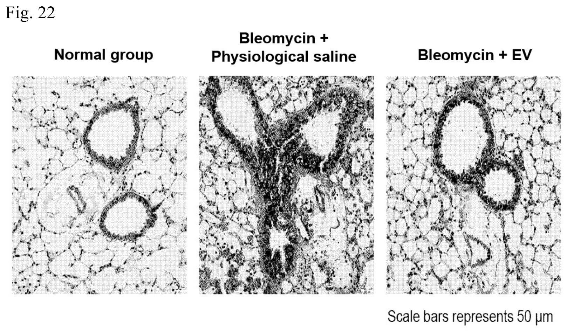

As a result, as shown in Figure 22, it was confirmed that in the bleomycin (BLM) administration group, a stained collagen area was present over a wide area. On the other hand, in the BLM+hMSCs-EV administration group, it was confirmed that the degree of collagen deposition in lung tissue was reduced to a level similar to that of the normal group.

### Example 6.2. Evaluation of therapeutic efficacy for pulmonary fibrosis in an animal model of pulmonary fibrosis produced by intratracheal administration of bleomycin

### Example 6.2.1. Construction of animal model of pulmonary fibrosis and administration of extracellular vesicles

In order to confirm the therapeutic effect of the extracellular vesicles obtained according to the method described in Example 1 above on pulmonary fibrosis, an animal model of pulmonary fibrosis was produced, and the therapeutic effect of the extracellular vesicles on pulmonary fibrosis was evaluated in the produced pulmonary fibrosis animal model.

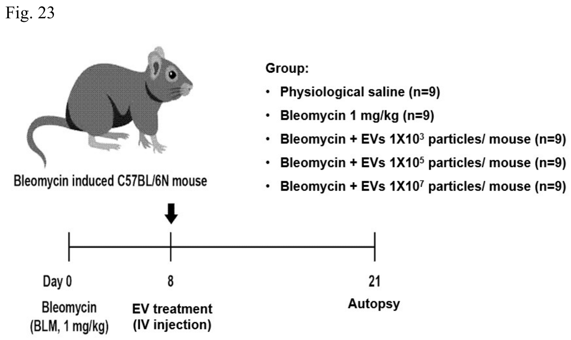

Specifically, 8-week-old male C57BL/6N mice were intratracheally administered with bleomycin (BLM), known to induce pulmonary fibrosis, at a concentration of 1 mg/kg (100 µL) once daily to produce a pulmonary fibrosis model (Figure 23). The extracellular vesicles to be used in the experiment were prepared by dispersing the extracellular vesicles obtained according to the method described in Example 1 above in saline solution so that they could be administered at doses of 1 x 10³, 1 x 10⁵, or 1 x 10⁷ particles per mouse, thereby preparing an injection.

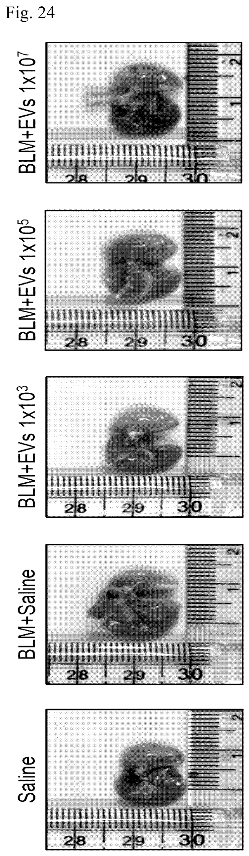

The extracellular vesicles (1 x 10³, 1 x 10⁵, and 1 x 10⁷ particles/mouse) prepared as described above were administered intravenously as a single dose of 100 µL per mouse to the pulmonary fibrosis model mice (day 8 after the start of bleomycin administration). During the administration and observation period, general symptoms and body weight were observed once a day and recorded for each animal. On day 13 after administration of extracellular vesicles (day 21 after the start of bleomycin administration), lung tissue was collected from the mice for morphological examination (Figure 24). The expression of fibrosis marker genes and proteins, as well as the soluble collagen assay and Masson's trichrome staining, were used to confirm the therapeutic effect of extracellular vesicles on pulmonary fibrosis.

### Example 6.2.2. Confirmation of changes in gene expression of fibrosis-related factors in lung tissue

Lung tissue was obtained from the experimental mice of Example 6.2.1 above, and the gene expression of fibrosis-related factors in lung tissue was confirmed using q-PCR.

Specifically, the right middle lobe of the lung tissue from the experimental mice of Example 6.2.1 above was extracted and pulverized using a homogenizer. Trizol solution (1 mL) was added to the pulverized tissue samples and reacted overnight at -80 °C. Then, RNA was extracted using an RNA extraction kit. After measuring the RNA concentration, cDNA was synthesized according to the manufacturer's protocol. The gene expression levels in each sample were confirmed through q-PCR using primers for COL1A1 (collagen type I alpha 1 chain), α-SMA, Timp, and fibronectin. At this time, the primer sequences used are shown in Table 1.

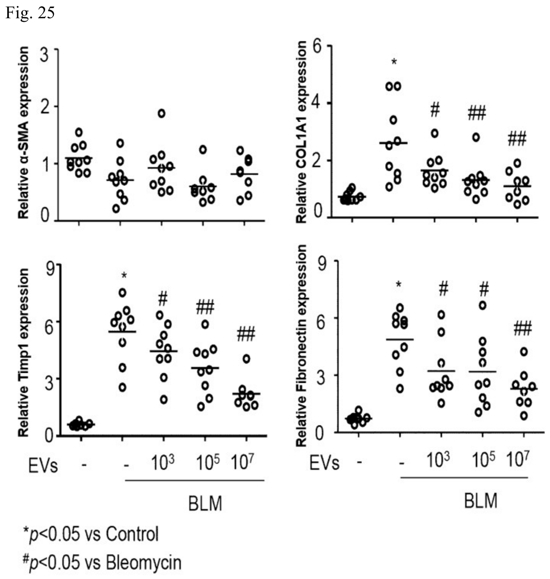

As a result, as shown in Figure 25, it was confirmed that the gene expression of COL1A1 (collagen type I alpha 1 chain), Timp, and fibronectin was increased by bleomycin (BLM) administration, and that the increased gene expression of the above genes was reduced by hMSCs-EV administration.

### Example 6.2.3. Confirmation of expression of fibrosis-related proteins in lung tissue

The right middle lobe of the lung obtained from the experimental mice of Example 6.2.1 above was extracted and pulverized using RIPA buffer and a homogenizer. The pulverization was performed for 20 minutes at 2-8 °C, and the supernatant protein was isolated and used by centrifugation at 13,000 rpm for 15 minutes.

The protein samples were subjected to Western blotting using the same method as in Example 4.4 above to confirm the expression of fibrosis-related proteins. At this time, the primary antibodies used were anti-Timp1 antibody (Cell Signaling, #63363), anti-α-SMA antibody (Abcam, ab5694), anti-Col1a1 antibody (Abcam, ab260043), anti-fibronectin antibody (Abcam, ab2413), and anti-GAPDH antibody (Cell Signaling, #2118). The secondary antibody used was anti-rabbit IgG, HRP-linked antibody (Cell Signaling, 7074S).

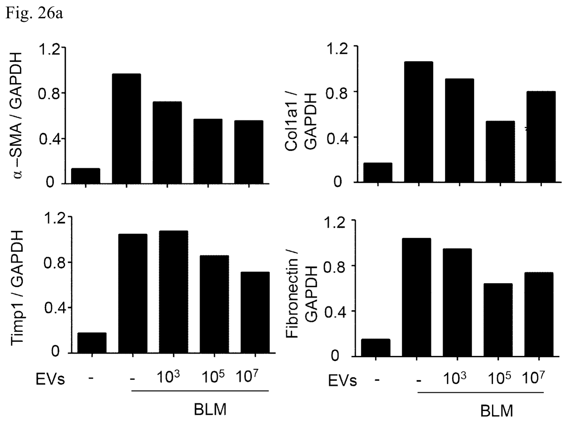

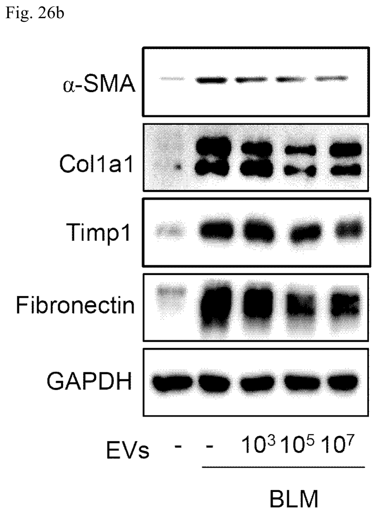

As a result, as shown in Figures 26a and 26b, it was confirmed that the expression of fibrosis-related proteins (α-SMA, COL1A1, Timp1, and fibronectin) was reduced in the BLM+hMSCs-EV administration group compared to bleomycin (BLM) administration group.

### Example 6.2.4. Confirmation of soluble collagen concentration in lung tissue

The inferior and superior lobes of the lungs obtained from the experimental mice of Example 6.2.1 above were extracted and pulverized using a homogenizer. The soluble collagen concentration in the tissues was measured using the same method as in Example 6.1.3 above.

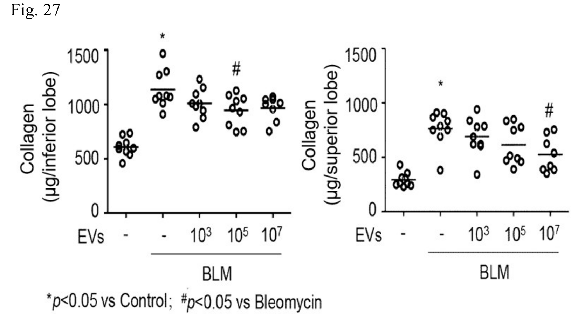

As a result, as shown in Figure 27, it was confirmed that the collagen concentration in both the inferior and superior lobes was reduced in the BLM+hMSCs-EV administration group compared to the bleomycin (BLM) administration group.

### Example 6.2.5. Confirmation of pathological morphology of lung tissue through hematoxylin and eosin staining

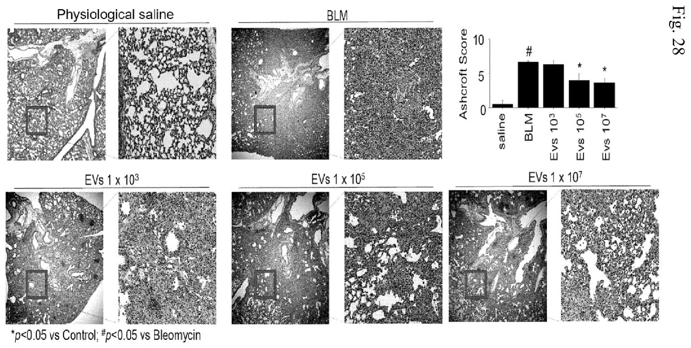

The left lobe of the lung obtained from the experimental mice of Example 6.2.1 above was extracted, and tissue sections were prepared using the same method as in Example 6.1.4 above and stained with hematoxylin and eosin. At this time, the tissue sections were treated with Weigert's iron hematoxylin working solution, stained with Eosin working solution for 30 seconds, and then washed in running water for 3 minutes. Thereafter, dehydration (ethanol 70% (2 minutes), ethanol 80% (2 minutes), ethanol 90% (2 minutes), ethanol 100% (2 minutes), xylene (3 minutes)) was performed, and then the sections were mounted, and the pathological morphology of the lung tissue was confirmed under a microscope. In addition, the observation results were expressed as Ashcroft scores (Figure 28).

### Example 6.2.6. Confirmation of collagen expression areas in lung tissue through Masson's trichrome staining

The collagen expression areas in the lung tissue obtained from the experimental mice of Example 6.2.1 above were confirmed through Masson's trichrome staining using the same method as in Example 6.1.4 above.

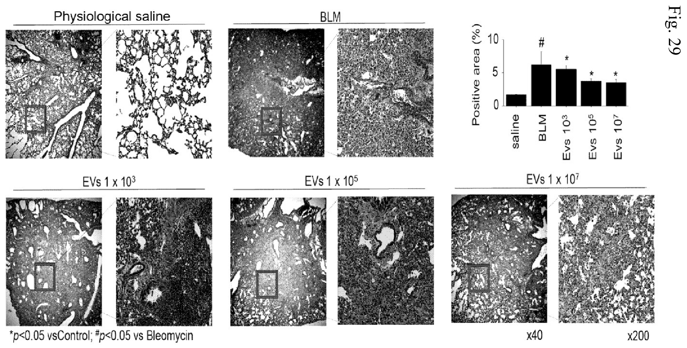

As a result, as shown in Figure 29, it was confirmed that the collagen area in the lung tissue was significantly reduced in the BLM+hMSCs-EV administration group compared to the bleomycin (BLM) administration group, and the level was similar to that of the normal group.

Through the above results, it was confirmed that the extracellular vesicles isolated from human mesenchymal stem cells have the effect of improving or treating pulmonary fibrosis.

## Claims

1. An extracellular vesicle comprising any one overexpressed microRNA selected from the group consisting of hsa-miR-148a-3p, hsa-miR-100-5p, hsa-miR-143-3p, hsa-let-7a-5p, hsa-let-7f-5p, and a combination thereof.

2. The extracellular vesicle according to claim 1, wherein the extracellular vesicle comprises hsa-miR-148a-3p at 1 to 20% of the total microRNA content of the extracellular vesicle.

3. The extracellular vesicle according to claim 1, wherein the extracellular vesicle comprises hsa-miR-100-5p at 1 to 20% of the total microRNA content of the extracellular vesicle.

4. The extracellular vesicle according to claim 1, wherein the extracellular vesicle comprises hsa-miR-143-3p at 0.1 to 10% of the total microRNA content of the extracellular vesicle.

5. The extracellular vesicle according to claim 1, wherein the extracellular vesicle comprises hsa-let-7a-5p at 0.1 to 10% of the total microRNA content of the extracellular vesicle.

6. The extracellular vesicle according to claim 1, wherein the extracellular vesicle comprises hsa-let-7f-5p at 0.1 to 10% of the total microRNA content of the extracellular vesicle.

7. The extracellular vesicle according to claim 1, wherein the microRNA exhibits the fibrosis inhibition activity.

8. The extracellular vesicle according to claim 1, wherein the extracellular vesicle further comprise any one expressed microRNA selected from the group consisting of hsa-miR-151a-3p, hsa-let-7b-5p, hsa-miR-21-5p, hsa-miR-10a-5p, hsa-miR-92a-3p, hsa-miR-486-5p, hsa-miR-99b-5p, hsa-let-7i-5p, hsa-miR-320a, hsa-miR-409-3p, hsa-miR-10b-5p, hsa-miR-127-3p, hsa-miR-26a-5p, hsa-miR-221-3p, hsa-let-7e-5p, hsa-miR-493-5p, hsa-miR-25-3p, hsa-miR-574-3p, hsa-miR-423-5p, hsa-miR-122-5p, hsa-miR-382-5p, hsa-miR-155-5p, hsa-miR-451a, hsa-miR-30a-3p, hsa-miR-28-3p, hsa-let-7g-5p, hsa-miR-379-5p, hsa-miR-23a-3p, hsa-miR-485-5p, hsa-miR-30a-5p, hsa-miR-30d-5p, hsa-miR-423-3p, hsa-miR-99a-5p, hsa-let-7c-5p, hsa-miR-92b-3p, hsa-miR-323a-3p, hsa-miR-574-5p, hsa-miR-197-3p, hsa-miR-432-5p, hsa-miR-140-3p, hsa-miR-370-3p, hsa-miR-196a-5p, hsa-miR-654-5p, hsa-miR-184, hsa-miR-181a-2-3p, and a combination thereof.

9. The extracellular vesicle according to claim 1, wherein the extracellular vesicle is isolated from a human-derived stem cell.

10. The extracellular vesicle according to claim 9, wherein the stem cell is a mesenchymal stem cell.

11. The extracellular vesicle according to claim 10, wherein the mesenchymal stem cell is derived from umbilical cord, umbilical cord blood, bone marrow, fat, muscle, nerve, skin, amniotic membrane, tooth, hair root cell, or placenta, or is differentiated from an induced pluripotent stem cell.

12. The extracellular vesicle according to claim 1, wherein the particle size of the extracellular vesicle is 10 nm to 1,000 nm.

13. A pharmaceutical composition for preventing or treating fibrosis, comprising the extracellular vesicle according to claim 1 as an active ingredient.

14. The pharmaceutical composition for preventing or treating fibrosis according to claim 13, wherein the fibrosis is pulmonary fibrosis.

15. The pharmaceutical composition for preventing or treating fibrosis according to claim 13, wherein the pulmonary fibrosis is any one or more selected from the group consisting of idiopathic pulmonary fibrosis, radiation-induced lung injury, nonspecific interstitial pneumonia, acute interstitial pneumonia, cryptogenic organizing pneumonia, respiratory bronchiolitis-associated interstitial lung disease, desquamative interstitial pneumonia, lymphoid interstitial pneumonia, interstitial pulmonary fibrosis and diffuse pulmonary fibrosis, pulmonary edema, cystic fibrosis, and pulmonary fibrosis caused by metabolic diseases.

16. A use of the extracellular vesicle according to claim 1 for the prevention or treatment of fibrosis.

17. A method for preventing or treating fibrosis, comprising administering the extracellular vesicle according to claim 1 to a subject.
